# FASCICULE DE BREVET EUROPEEN

(11) **EP 2 758 391 B1**
(45) Date de publication et mention de la délivrance du brevet: **15.03.2017**
(21) Numéro de dépôt: 12775749.0
(22) Date de dépôt: 20.09.2012
(51) Int. Cl.: C07D 401/14, C07D 497/04, G01N 33/533, C07F 9/6558

(54) **SONDES LUMINESCENTES POUR LE MARQUAGE BIOLOGIQUE ET L'IMAGERIE, ET LEUR PROCÉDÉ DE PRÉPARATION.**
LUMINESZIERENDE SONDEN ZUR BIOLOGISCHEN MARKIERUNG UND BILDGEBUNG SOWIE HERSTELLUNGSVERFAHREN DAFÜR
LUMINESCENT PROBES FOR BIOLOGICAL MARKING AND IMAGERY, AND PREPARATION METHOD THEREOF

(30) Priorité: 22.09.2011 FR 1158433
(43) Date de publication de la demande: 30.07.2014
(73) Titulaire: Centre National de la Recherche Scientifique, 75016 Paris (FR)
(72) Inventeur: ZIESSEL, Raymond, F-67460 Souffelweyersheim (FR); STARCK, Mathieu, F-67200 Strasbourg (FR); SUTTER, Alexandra, F-68310 Wittelsheim (FR)
(74) Mandataire: Goulard, Sophie
(86) Numéro de dépôt international: PCT/FR2012/052104
(87) Numéro de publication internationale: WO 2013/041811

(56) Documents cités:
- FR-A1- 2 935 973
- MATTHIEU STARCK ET AL: "Towards Libraries of Luminescent Lanthanide Complexes and Labels from Generic Synthons", CHEMISTRY - A EUROPEAN JOURNAL, vol. 17, no. 33, 8 août 2011 (2011-08-08), pages 9164-9179, XP055024807, ISSN: 0947-6539, DOI: 10.1002/chem.201100390
- ANTHONY D'ALÉO ET AL: "Charge Transfer Excited States Sensitization of Lanthanide Emitting from the Visible to the Near-Infra-red", COORDINATION CHEMISTRY REVIEWS, 1 mars 2012 (2012-03-01), XP055024815, ISSN: 0010-8545, DOI: 10.1016/j.ccr.2012.03.023

## Description

La présente invention concerne des composés organiques, utilisables comme ligands pour la préparation de complexes de lanthanides ou de certains métaux de transition solubles dans l'eau, un procédé pour leur préparation, ainsi que leur utilisation à titre de sonde fluorescente.

Les complexes d'ions lanthanides possèdent des propriétés spectroscopiques très particulières qui permettent des applications dans le domaine de la détection par luminescence. Ces complexes possèdent un très large déplacement de Stokes, des raies d'émission très fines, de l'ordre de quelques nm, qui sont caractéristiques de l'ion lanthanide utilisé. Ils peuvent émettre dans le domaine visible ou proche infrarouge, et ils ont un temps de vie de l'état excité extrêmement long, pouvant atteindre la milliseconde. Cette dernière caractéristique est un atout essentiel : elle permet une détection en temps résolu (ce qui permet d'éliminer les signaux de fluorescence parasite) et elle provoque une très forte augmentation de la sensibilité de détection des complexes en microscopie de luminescence ou dans les analyses fluoroimmunologiques. Les complexes luminescents d'ions lanthanides ont par conséquent des applications dans la plupart des domaines de la fluorescence conventionnelle.

Cependant, les complexes d'ions lanthanides sont généralement difficiles à obtenir. De nombreuses propriétés du complexe dépendent de la structure du ligand et de l'ion lanthanide, notamment l'efficacité d'excitation du complexe, le degré de stabilité de la complexation des lanthanides en milieu chimique compétitif et en milieu sérique (qui doit être élevé pour éviter le relargage des cations), le rendement quantique de luminescence et la possibilité de former des liaisons covalentes avec le matériel à marquer pour les applications biologiques des complexes. Une excitation adéquate des complexes peut être obtenue lorsque le ligand du complexe comprend des groupements hétéroaromatiques qui ont pour but de capter la lumière et de la transférer à l'ion lanthanide qui va réémettre. Ce phénomène est appelé effet d'antenne. Le choix de ces groupements hétéroaromatiques définit de nombreuses propriétés spectroscopiques du complexe final, notamment la gamme spectrale d'excitation et le rendement quantique de luminescence.
CN-1811429-A décrit un complexe de Tb³⁺ et d'un ligand qui a un squelette 2,6-dipyrazolylpyridine dans lequel chacun des groupes pyrazolyle porte un groupement -CH₂-N(CH₂CO₂H)₂. Ledit complexe est utile pour la détection de l'oxygène singulet. Il est obtenu par un procédé consistant à fixer le groupe anthracène sur une dibromo-aminopyridine, puis à modifier le groupe pyridyle par réaction des atomes de brome avec les réactifs appropriés pour remplacer chaque Br par un groupe pyrazolyle portant un groupe -CH₂-N(CH₂CO₂H)₂.
EP-0 770 610 décrit des complexes d'ion lanthanide dans lesquels le ligand est un squelette 2,6-dipyrazolylpyridine dans lequel chacun des groupes pyrazolyle porte un groupement -CH₂-N(CH₂CO₂H)₂. Le procédé de préparation consiste à préparer d'abord un composé 2,6-dipyrazolylpyridine di bromé, puis on le modifie pour obtenir les deux groupes terminaux -CH₂-N(CHG₂CO₂H)₂. Ce procédé ne permet pas d'obtenir des composés dans lesquels le groupe pyridyle porte des substituants choisis pour ajuster les propriétés du complexe de lanthanide dans lesquels lesdits composés constituent le ligand.
FR-2 935 973 décrit des ligands dérivés de 2,6-dipyrazolylpyridine dans lesquels chacun des groupes pyrazolyle porte un groupement -CH₂-N(CHG₂CO₂R)₂ dans lequel chacun des R représente H ou un métal alcalin ou un groupe ammonium quaternaire, les groupes pyrazolyle pouvant en outre porter un ou deux substituants choisis parmi un groupe alkyle ayant de 1 à 4 atomes de carbone, ou bien ces deux substituants forment ensemble un biradical formant un cycle aromatique avec les deux atomes de carbone qui les portent. Ces ligands sont aptes à complexer les ions lanthanide et trouvent des applications pour le marquage et la microscopie biphotonique. Ils présentent cependant une faible solubilité dans l'eau et une grande instabilité en milieu purement aqueux, en milieu salin ou en milieu purement biologique.

Le but de la présente invention est de fournir des composés utiles comme ligands pour des complexes de lanthanide ou de métal de transition qui aient une solubilité améliorée dans l'eau et dans les milieux biologiques, ainsi qu'une excellente stabilité chimique, de très bonnes propriétés de luminescence et des temps de vie très longs.

Ce but est atteint par les composés qui font l'objet de la présente invention et qui vont être décrits ci-après.

Un composé selon la présente invention répond à la formule (I) suivante : 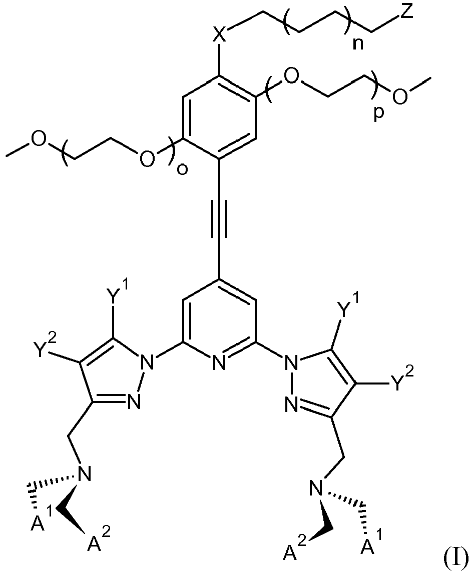 dans laquelle :
- n, o, p, indépendamment les uns des autres, sont des nombres entiers variant de0à4;
- A¹ représente une fonction -COOR¹ dans laquelle R¹ est un atome d'hydrogène ou un cation alcalin ; ou un groupement A² ;
- A² représente un groupement -P(O)(OCH₂CH₃)(OR²) ou -P(O)(OR²)₂ dans lesquels R² représente un atome d'hydrogène ou cation alcalin ;
- chacun des Y¹, Y² représente un atome d'hydrogène ou bien Y¹ et Y² forment ensemble un biradical formant un ou plusieurs cycles aromatiques ou hétéroaromatique avec les deux atomes de carbone qui les portent, lesdits cycles aromatiques portant éventuellement un ou plusieurs substituants choisis parmi un atome d'hydrogène, un groupement amino et un groupement thiol,
- X représente un segment de liaison constitué par une fonction amide -C(O)-NH- ;
- Z est :
   * NH₂, un halogène, un phosphate,
   * un groupe COOR³ dans lequel R³ est H, un cation alcalin, un groupe ammonium quaternaire N(R⁴)₄⁺ dans lequel R⁴ est H ou une chaîne alkyle linéaire ayant de préférence de 1 à 4 atomes de carbone, un groupe succinimide -N-(CO-CH₂-CH₂-CO)- ou un groupe pentafluorophényle -C₆F₅ ;
   * une fonction biotine (-CO-(CH₂)₄-C₅H₇N₂OS), un groupement polyhétéroaromatique ou un éther couronne,
   * un cluster organique ou inorganique silylé ;
   * un support macroscopique tel que par exemple une bille de silice, une bille de nanolatex ou tout autre support susceptible d'être fonctionnalisé.

Parmi les cations alcalins mentionnés pour R¹ et R², on peut en particulier citer K⁺, Na⁺ et Li⁺.

Les composés de formule (I) dans lesquels les groupes Y¹ et Y² font partie d'un cycle aromatique ou hétéroaromatique sont particulièrement préférés.

Un composé de formule (I) selon la présente invention peut être préparé à partir d'un composé répondant à l'une des formules (V) et (V') suivantes : 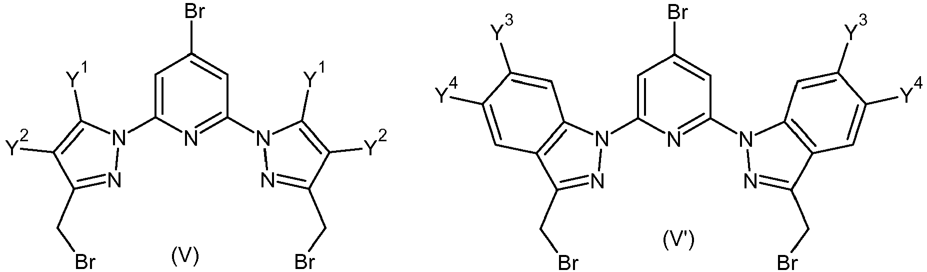 dans lesquelles les groupements Y¹, Y², ont la même signification que celle indiquée ci-dessus pour les composés de formule (I) et les groupements Y³ et Y⁴, identiques ou différents, sont choisis parmi un atome d'hydrogène, un groupement amino et un groupement thiol.

Les composés de formule (V) et (V') peuvent être préparés selon le procédé décrit dans la demande de brevet FR-2 935 973.

De manière plus précise, et à titre d'exemple, les composés de formule (I), peuvent être préparés selon un procédé comprenant les étapes suivantes :
i) une première étape de substitution nucléophile des atomes de brome d'un composé de formule (V) ou (V'), dans un solvant anhydre tel que l'acétonitrile anhydre, en présence d'une base minérale choisie par exemple parmi K₂CO₃, Na₂CO₃ et Cs₂CO₃ et d'un composé choisi parmi les composés de formule (III) suivante [NH(CH₂COOR'¹)(CH₂P(O)(OR'²)₂] et les composés de formule (IV) suivante [NH{CH₂P(O)(OR'²)₂}₂] dans lesquelles R'¹ et R'², indépendamment l'un de l'autre, représentent un radical alkyle linéaire ou ramifié en C₁-C₄, ladite substitution nucléophile étant réalisée à une température comprise entre environ 60°C et 80°C pour obtenir un composé de formule (VI), respectivement de formule (VI') suivantes : 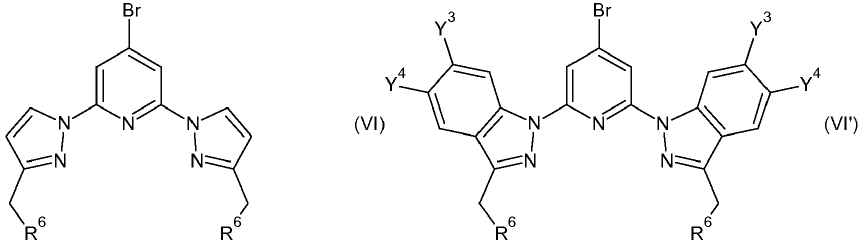 dans lesquelles R⁶ est choisi parmi les groupements de formule R⁶-a ou R⁶-b suivants : 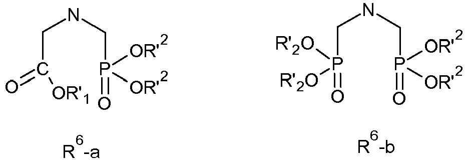 dans lesquels :
   - R'¹ représente un radical alkyle linéaire ou ramifié en C₁-C₄ tel que par exemple un radical éthyle ou ter-butyle ;
   - R'² représente un radical C₁-C₄ tel que par exemple un radical éthyle ;
ii) une deuxième étape de préparation d'un composé 4 de formule suivante : 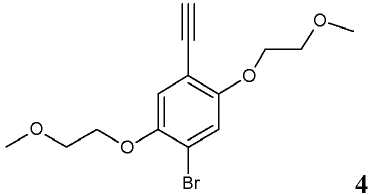 selon un procédé comprenant les sous-étapes suivantes :
   ii-a) la mise en réaction du paradiphénol avec du tosylate de 2-méthoxyéthanol, dans un solvant anhydre tel que l'acétonitrile anhydre en présence d'une base minérale choisie par exemple parmi K₂CO₃, Na₂CO₃ et Cs₂CO₃, à une température d'environ 80°C pendant 12 heures environ pour obtenir un composé 1 de formule suivante : 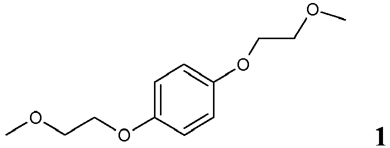
   ii-b) une étape de bromation du composé **1** obtenu à l'étape précédente par du brome (Br₂), dans un solvant tel que le tétrachlorométhane au reflux pendant environ 12 heures pour obtenir un composé **2** de formule suivante : 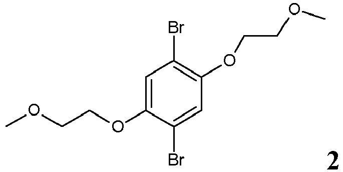
   ii-c) une étape de substitution d'un des deux atomes de brome du composé **2** obtenu à l'étape précédente, dans un solvant tel que le tetrahydrofurane (THF), en présence de di-isopropylamine, d'un réactif (CH₃)₂C(OH)C≡CH et d'un catalyseur au palladium tel qu'un complexe de palladium et de triphénylphosphine, en particulier Pd(PPh₃)₂Cl₂, et de CuI, pour obtenir un composé 3 de formule suivante : 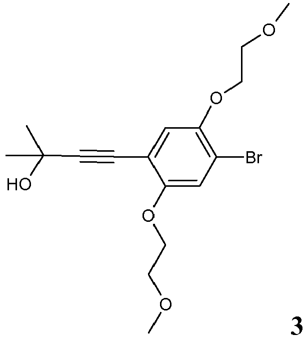
   ii-d) une étape d'hydrolyse alcaline du composé 3 obtenu ci-dessus à l'étape précédente par une base forte anhydre telle que NaOH anhydre, dans un solvant organique tel que le toluène à une température d'environ 130°C pendant 12 heures environ, pour obtenir ledit composé 4 ;
iii) une troisième étape de couplage croisé entre le composé 4 obtenu ci-dessus à l'étape ii) et le composé de formule (VI), respectivement (VI') obtenu ci-dessus à l'étape i), dans un solvant organique en présence d'un catalyseur au palladium et de triphénylphosphine, en particulier Pd(PPh₃)₂Cl₂, pour obtenir un composé de formule (VII), respectivement (VII'), suivantes : 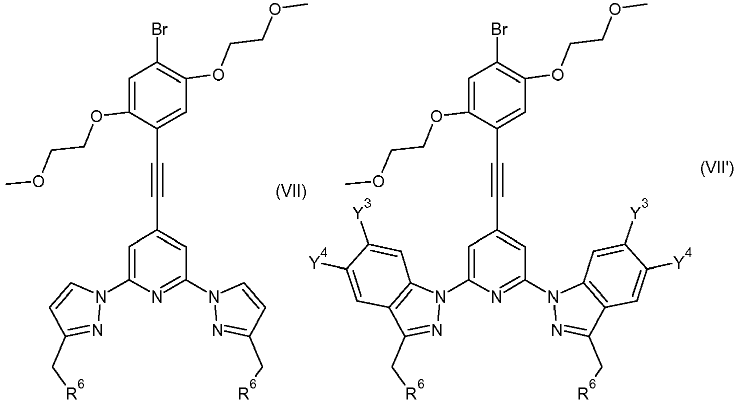 dans lesquelles R⁶ a la même signification que dans les composés de formule (VI), respectivement (VI'), ci-dessus ;
iv) une quatrième étape au cours de laquelle le brome du composé de formule (VII), respectivement (VII') est remplacé par un groupe de formule X-CH₂-(CH₂CH₂)ₙ-CH₂-Z dans lequel n, X et Z ont la même signification que celle indiquée ci-dessus relativement aux composés de formule (I), par réaction avec un réactif approprié, en présence d'un catalyseur organométallique, dans un solvant organique ;
v) une cinquième étape au cours de laquelle les groupes R'¹ et R'² sont respectivement remplacés par des groupes R¹ et R², lesdits groupements étant H ou un cation alcalin (pour R¹) ou un cation alcalin (pour R²).

Selon une forme de réalisation préférée du procédé de préparation des composés de formule (I), l'étape iv) de substitution de l'atome de brome par le groupement de formule X-CH₂-(CH₂CH₂)ₙ-CH₂-Z est réalisée dans les conditions de couplage croisé de type Sonogashira ou de carboamidation catalysée par du palladium sous-ligandé.

Lors de la cinquième étape, le remplacement des groupes R'¹ et R'² du type alkyle linéaire par un cation K⁺, Na⁺ ou Li⁺ peut être effectué avec KOH, NaOH ou LiOH respectivement dans un solvant polaire (par exemple un mélange CH₃OH/H₂O), à une température entre 20 et 100°C, par exemple à 60°C, ou bien en utilisant du bromure de triméthylsilyle dans un solvant tel que le dichlorométhane à température ambiante suivi d'une hydrolyse basique. Le groupe COOK, COONa ou COOLi peut ensuite, si nécessaire, être modifié en groupe acide par réaction avec un acide tel que HCl dans l'eau.

Egalement lors de la cinquième étape, le remplacement par H de groupes R'¹ ou R'² du type alkyle ramifié peut être effectué par réaction avec l'acide trifluoroacétique (TFA) dans un solvant organique aprotique.

Quelques exemples de ligands, auxquels l'invention n'est cependant pas limitée, sont donnés ci-après à titre purement illustratif. Des ligands similaires peuvent être obtenus en remplaçant Na par l'un des autres groupes R¹ ou R² tels que définis précédemment.

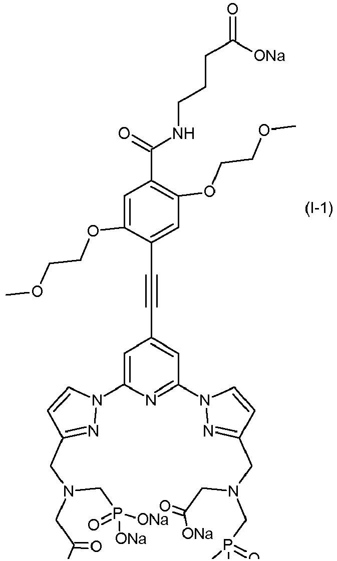

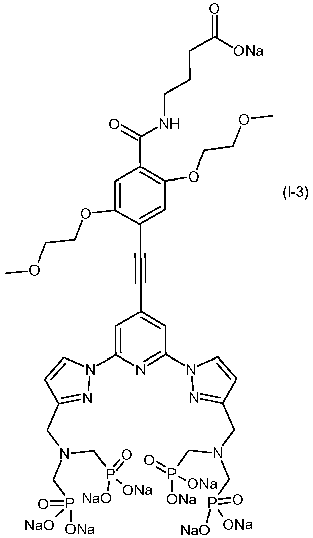

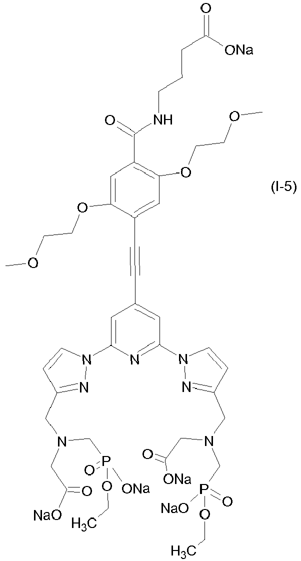

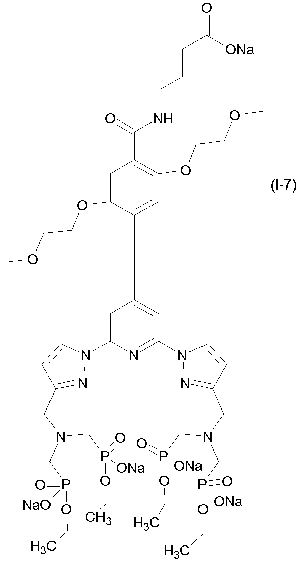

Un complexe de lanthanide ou d'un métal de transition selon l'invention comprend un ion lanthanide ou d'un métal de transition, complexé par un ligand de formule (I) tel que défini précédemment. L'ion lanthanide est choisi parmi les ions Gd³⁺, Lu³⁺, Eu³⁺, Tb³⁺, Dy³⁺, Sm³⁺, Er³⁺, Yb³⁺, Pr³⁺ et Nd³⁺. L'ion d'un métal de transition peut être choisi parmi les métaux suivants Cu(II), Co(II), Mn(II ou IV), Ni(II), Fe(III), Pd(II) et Pt(II).

Un complexe de lanthanide ou de métal de transition peut être obtenu par mélange de quantités équimoléculaires d'un composé de formule (I) (de préférence sous forme de sel de sodium) avec un sel de lanthanide, respectivement un sel de métal de transition, par chauffage du mélange, refroidissement et neutralisation à pH de 6 à 8, puis récupération du complexe à la température ambiante.

Le sel de lanthanide peut être un nitrate, un chlorure, un perchlorate ou un triflate Ln(CF₃SO₃)₃.

Le sel de métal de transition peut par exemple être un chlorure, acétate, nitrate ou tout autre composé soluble dans l'eau ou les alcools.

Dans un mode de réalisation particulier, le composé de formule (I) et le sel de lanthanide ou le sel de métal de transition sont mélangés en quantités équimolaires en solution dans l'eau ou dans un mélange MeOH/eau, la solution est chauffée pendant 2 à 3 heures à 60°C, refroidie à température ambiante puis neutralisée si nécessaire à pH 7 par addition de NaOH, d'un hydroxyde d'ammonium quaternaire ou de HCl dilué dans l'eau. Les complexes formés sont ensuite isolés par concentration des eaux mères et précipitation dans un mélange H₂O/MeOH/THF/Et₂O.

Les complexes selon l'invention ont des applications variées, selon la nature des substituants du groupement pyridyle du ligand. Leurs propriétés optiques de luminescence, les temps de vie de luminescence et les propriétés de brillance sont exceptionnelles avec des rendements quantiques quasi-quantitatifs (**Tb(I-5)** et **Tb(I-7)**) et des temps de vie des états excités supérieurs à 3 millisecondes dans la plupart du temps.

A titre d'exemple, l'ensemble des propriétés d'absorption et d'émission des complexes de terbium synthétisés à partir des ligands (I-1) et (I-3) conformes à
l'invention est résumé dans le Tableau 1 suivant :

**TABLEAU 1**

| Nature du Complexe | Absorption^{a} | | Emission | | | |
|---|---|---|---|---|---|---|
| | λ_{abs} (nm) | ε (M⁻¹.cm⁻¹) | τ (ms)^{b} | | φ (%)^{b} | |
| | | | H₂O | D₂O | H₂O | D₂O |
| **Tb(I-5)** | 283 | 45600 | 2,6 | 3,1 | 88 | 96 |
| | 328 | 10900 | | | | |
| **Tb(I-7)** | 284 | 44300 | 3,1 | 3,4 | 86 | 94 |
| | 328 | 10200 | | | | |

| | | | | | | |
|---|---|---|---|---|---|---|
| a) Dans un tampon 0,01 M TRIS/HCl à pH = 7.0. b) Erreur estimée à ± 15 % sur le rendement quantique et ± 5 % sur le temps de vie τ. | | | | | | |

Les paramètres φ et τ représentent respectivement les rendements quantiques de luminescence et les temps de vie de luminescence. Ils peuvent être mesurés dans l'eau et dans D₂O.

Dans une configuration préférée de l'invention plusieurs tests de stabilité montrent l'absence de dégradation de la sonde fluorescente. Il a été démontré en particulier qu'aucune perte de luminescence des complexes de terbium à λₘₐₓ 490 et 543 nm et d'europium à λₘₐₓ 590 et 620 nm n'a été observée sur plusieurs jours lorsque ces complexes sont dissous en milieu aqueux à pH neutre ou en milieu salins à des concentration de 10⁻⁴ à 10⁻⁶ M.

A titre d'exemple, la stabilité des complexes de terbium synthétisés à partir des ligands (I-1) et (I-3) conformes à l'invention dans différents tampons, est donnée dans le Tableau 2 ci-dessous. A titre de comparaison, le Tableau 2 donne également la stabilité d'un complexe de terbium TbL4 de l'art antérieur et répondant à la formule suivante :

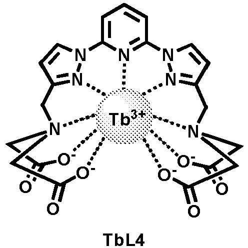

**TABLEAU 2**

| Tampons | τ (ms) | | |
|---|---|---|---|
| | Erreur estimée ± 5 % sur τ | | |
| | **TbL4 *** | **Tb(I-5)** | **Tb(I-7)** |
| H₂O | 2,8 | 2,6 | 3,1 |
| Phosphate 0,1 M, pH = 7,0 | 2,8 | 2,6 | 3,1 |
| Tris 0,1 M, pH = 7,0 | 2,8 | 2,6 | 3,0 |
| Tris 0,1 M - EDTA 10 mM, pH = 7,0 | 0,6 | 2,6 | 3,0 |
| Tris 50 mM - Sérum 50%, pH = 7,0 | 0,2 | 2,5 | 3,1 |
| Tris 0,1 M - KF 400 mM, pH = 7,0 | 0 | 2,5 | 3,0 |

| | | | |
|---|---|---|---|
| * Complexe non conforme à l'invention | | | |

Cette étude de stabilité montre que les complexes conformes à l'invention présentent une excellente stabilité avec des temps de vie supérieurs à 3 millisecondes dans divers milieux aqueux.

Par ailleurs, les complexes conformes à l'invention ont une solubilité dans l'eau exceptionnelle. Leur solubilité varie de 1 à 10 milligramme par millilitre de tampon phosphate.

De plus, les coûts de production et la purification de ces composés en font des candidats potentiels très intéressants pour l'analyse biomédicale et biologique en temps résolu pour quantifier des traces d'analytes en excitant spécifiquement le ligand qui est considéré comme l'antenne collectrice de photons. Les composés conformes à l'invention permettent d'accommoder l'ensemble des lanthanides et les propriétés d'absorption des ligands sont modulables en fonction de la décoration apportée par le dit ligand.

En particulier, les complexes de invention sont utiles pour le marquage de composés qui portent un groupe amine, alcool, thiol, acide carboxylique ou ester activé. Ces composés peuvent être des molécules monomères ou des polymères éventuellement sous forme de billes. Pour cette utilisation, le groupe Z du ligand du complexe est de préférence un groupe -COOH, un groupe -COONa, un ester activé de type ester de *N*-Hydroxysuccinimide ou ester de pentafluorophénol, un Br, un I, ou une aminé.

Lorsque le ligand d'un complexe luminescent possède un site de reconnaissance électroniquement relié à la structure de complexation du ligand, les propriétés de luminescence du complexe peuvent être perturbées par la présence d'un analyte qui se fixe sur le site de reconnaissance. En couplant électroniquement le site de reconnaissance à la pyridine centrale par des liaisons acétyléniques, l'interaction du site de reconnaissance avec ses substrats induit des perturbations électroniques qui se traduisent par des changements de luminescence des complexes d'ion lanthanide.

La spectroscopie et la microscopie d'absorption à deux photons sont des techniques relativement récentes et qui ont pour intérêt, notamment en microscopie, de permettre une amélioration significative de la résolution spatiale en utilisant des photons de basses énergies. La quasi-totalité des marqueurs luminescents étudiés en absorption à deux photons sont des composés organiques fluorescents de type Rhodamine®. Ces composés organiques présentent de très faibles déplacements de Stokes et des bandes d'émission larges (correspondant à une largeur à mi-hauteur supérieure à 50 nm). Les complexes luminescents d'ions lanthanides selon la présente invention présentent des déplacements de Stokes très élevés et des bandes d'émission étroites. Ils constituent par conséquent des composés particulièrement intéressants pour le marquage et la microscopie biphotonique et des travaux très récents ont démontrés cette possibilité sur des complexes d'Eu et de Tb. [Cf. notamment a) Law, G.L.; et al. J. Am. Chem. Soc. 2008, 130, 3714 ; b) Picot, A.; et al., J. Am. Chem. Soc. 2008, 130, 1532.]. Les complexes de invention peuvent être utilisés comme marqueur luminescent pour l'absorption à deux photons. Pour cette application particulière, les ligands I-1, I-2, I-3 et I-4 seront préférentiellement utilisés.

Les complexes de l'invention dans lesquels le groupe terminal Z du ligand est un groupe biotine sont utiles pour la reconnaissance de l'avidine, de la streptavidine et de la neuravidine.

La présente invention est illustrée par les exemples suivants, auxquels elle n'est cependant pas limitée.

### EXEMPLE 1 :

### Synthèse du composé de formule (I-1)

Un composé de formule (I-1) a été synthétisé selon le schéma réactionnel suivant :

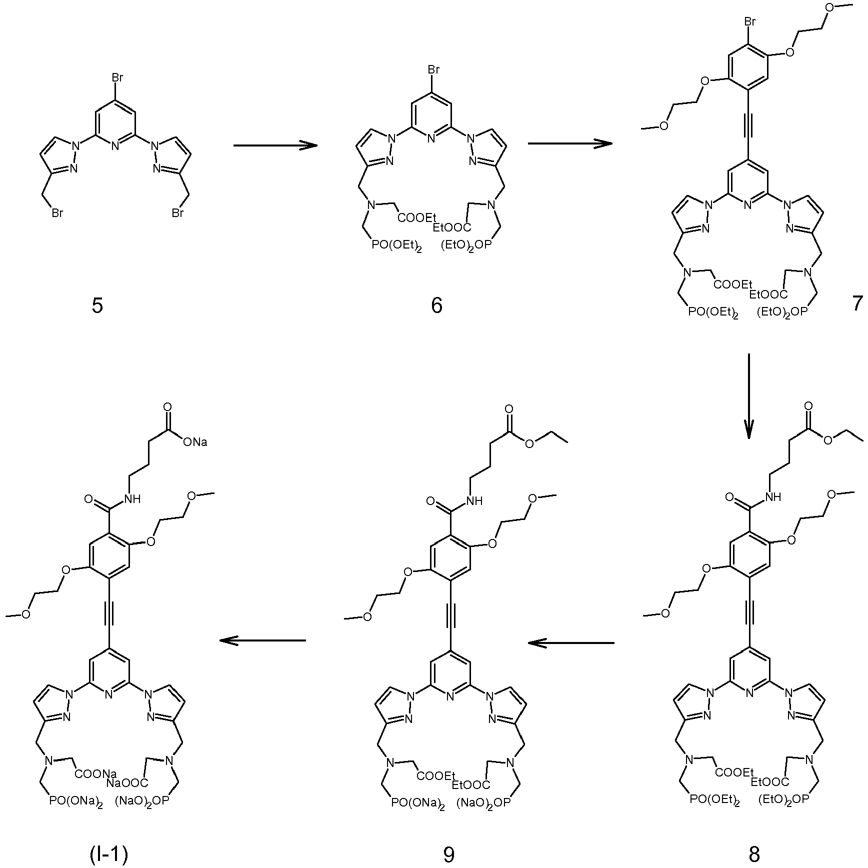

### 1) Première étape : Synthèse du composé 6

A une solution du composé 5 (préparé selon la référence P. Kadjane et al., Inorg. Chem., 2009, 48, 4601-4603) (267,6 mg, 0,562 mmol) dans de l'acétonitrile anhydre (10 mL), on a ajouté 270,5 mg de dérivé de glyphosate de formule NH[PO(OEt)₂(COOEt)] (préparé selon la référence S. Aime et al., Chem. Eur. J. 2006, 6, 2609-2617) (1,068 mmol) et 310,8 mg de K₂CO₃ (2,249 mmol). La solution résultante a été chauffée sous agitation à 60°C pendant 48 h sous argon. Le produit a été purifié par colonne de chromatographie sur silice en utilisant un mélange de solvants (de 0/10 à 2/8 (v/v) MeOH/CH₂Cl₂). On a obtenu le composé **6** attendu sous forme d'un solide blanc avec un rendement de 59 %.
¹H RMN (300 MHz, CDCl₃) δ (ppm) : 8,37 (d, J = 2,6 Hz, 2 H) ; 7,87 (s, 2 H) ; 6,47 (d, J = 2,6 Hz, 2 H) ; 4,02 - 4,18 (m, 12 H) ; 3,99 (s, 4 H) ; 3,58 (s, 4 H) ; 3,18 (s, 2 H) ; 3,14 (s, 2 H) ; 1,14 - 1,32 (m, 18 H).
³¹P {¹H} RMN (CDCl₃, 161 MHz) : δ 24,52 ppm.
SM-ESI : 821,1 (98), 819,1 (100).

Analyse élémentaire pour C₃₁H₄₈N₇O₁₀P₂Br :

| | **C** | **H** | **N** |
|---|---|---|---|
| **Calculé** | **45,37** | **5,90** | **11,95** |
| **Trouvé** | **45,09** | **5,76** | **11,76** |

### 2) Deuxième étape : Synthèse du composé 7

A une solution dégazée du composé **6** obtenu ci-dessus à l'étape précédente (100 mg, 0,122 mmol) dans un mélange benzène/triéthylamine (5 ml/1 ml) a été additionné 42,7 mg de composé **4** (0,130 mmol) et 10 mg de [Pd(PPh₃)₄]. La solution a été chauffée à 60°C durant une nuit et le composé **7** attendu a été isolé par chromatographie sur silice comme décrit ci-dessus à l'étape 1). Le composé **7** pur a été obtenu avec un rendement de 79% sous forme d'un solide blanc cristallin.
¹H RMN (300 MHz, CDCl₃) δ (ppm) : 8,35 (d, J = 2,6 Hz, 2 H) 7,84 (s, 2 H) ; 6,81 (s, 1H) ; 6,73 (s, 1H) ; 6,45 (d, J = 2,6 Hz, 2 H) ; 4,13 (m, 4H) ; 4,09 - 4,17 (m, 12 H) ; 4,04 (s, 4 H) ; 3,85 (m, 4H) ; 3,27 (s, 6H) ; 3,64 (s, 4 H) ; 3,23 (s, 2 H) ; 3,17 (s, 2 H) ; 1,14 - 1,32 (m, 18 H).
³¹P {¹H} RMN (CDCl₃, 161 MHz) : δ 24,47 ppm.
SM-ESI : 1069,2 (95), 1067,2 (100).

Analyse élémentaire pour C₄₅H₆₄N₇O₁₄P₂Br :

| | **C** | **H** | **N** |
|---|---|---|---|
| **Calculé** | **50,57** | **6,04** | **9,17** |
| **Trouvé** | **50,32** | **5,96** | **8,86** |

### 3) Troisième étape : Synthèse du composé 8

A une solution 115,0 mg de composé **7** obtenu ci-dessus à l'étape précédente (0,107 mmol) dans un mélange toluène (8 ml) / triéthylamine (8 ml), on a ajouté 51,7 mg de chlorhydrate de 4-éthylaminobutyrate (0,308 mmol) et 9,8 mg de Pd(PPh₃Cl₂) (0,014 mmol). La solution résultante a été chauffée à 70°C durant 12 h sous un flux continu de CO. Le composé **8** attendu a été obtenu sous forme d'un solide blanc après purification par chromatographie sur gel de silice avec un mélange de solvants comme éluant (de 0/10 à 4/6 MeOH/CH₂Cl₂). On a obtenu un rendement de 56 %.
¹H RMN (200 MHz, CDCl₃) δ (ppm) : 8,44 (d, J = 2,6 Hz, 2 H) ; 8,21 (s, 2H) ; 7,50 (m, 1 H) ; 6,80 (s, 1H) ; 6,70 (s, 1H) ; 6,49 (d, J = 2,6 Hz, 2H) ; 4,15 (m, 4H) ; 4,06 - 4,19 (m, 12H) ; 4,04 (s, 4H) ; 3,82 (m, 4H) ; 3,65 (s, 4 H) ; 3,52 (m, 4H) ; 3,27 (s, 6H) ; 3,24 (s, 2 H) ; 3,20 (s, 2 H) ; 2,43 (t, J = 7,0 Hz, 2 H) ; 1,98 (q, J = 7,0 Hz, 2 H) ; 1,15 - 1,37 (m, 21 H).
³¹P {¹H} RMN (CDCl₃, 161 MHz) δ 24,56 ppm.
SM-ESI: 1146,3 (100).

Analyse élémentaire pour C₅₂H₇₆N₈O₁₇P₂ :

| | **C** | **H** | **N** |
|---|---|---|---|
| **Calculé** | **54,44** | **6,68** | **9,77** |
| **Trouvé** | **54,13** | **6,43** | **9,56** |

### 4) Quatrième étape : Synthèse du composé 9

A une suspension aqueuse de 70,0 mg du composé **8** obtenu ci-dessus à l'étape précédente (0,061 mmol) dans 2 ml d'eau, on a ajouté 5 ml d'acide chlorhydrique 6N et chauffé le mélange résultant à 60°C durant 3 jours. Le composé **9** attendu a été obtenu par précipitation avec de l'éther et un rendement de 77%.
¹H RMN (200 MHz, MeOD + D₂O) δ (ppm) 8,80 (s, 2 H) ; 8,21 (d, J = 8,0 Hz, 2 H) ; 6,83 (s, 2 H) ; 6,76 (s, 1H) ; 6,65 (s, 1H) ; 4,85 (s, 4H) ; 4,39 (s, 4H) ; 4,09 (m, 4H) ; 3,78 (m, 4H) ; 3,74 - 3,96 (m, 5H) ; 3,26 (s, 6H) ; 3,18 (q, J = 7,0 Hz, 8H) ; 2,34 - 2,53 (m, 2 H) ; 1,93 (m, 2 H) ; 1,29 (t, J = 7,0 Hz, 9 H).
³¹P {¹H} RMN (CD₃OD, 161 MHz) : δ 8,70 ppm.
SM-ESI : 1035,4 (100).

Analyse élémentaire pour C₄₄H₆₀N₈O₁₇P₂, HCl :

| | **C** | **H** | **N** |
|---|---|---|---|
| **Calculé** | **50,57** | **5,89** | **9,82** |
| **Trouvé** | **50,16** | **5,56** | **9,65** |

### 5) Cinquième étape : Synthèse du composé (I-1)

A une solution de 40,9 mg du composé **9** (0,052 mmol) dans de l'eau (6 mL) à pH 7,0, on a ajouté 8,3 mg de NaOH (0,208 mmol) et on a agité la solution à température ambiante durant 24 heures. Le composé **(I-1)** attendu a été obtenu par précipitation à l'éther avec un rendement de 76 %.
¹H RMN (300 MHz, D₂O) δ (ppm) : 8,67 (s, 2H) 7,98 (d, J = 8,0 Hz, 2H) ; 6,75 (s, 2H) ; 6,76 (s, 1H) ; 6,65 (s, 1H) ; 4,81 (s, 4H) ; 4,32 (s, 4H) ; 4,11 (m, 4H) ; 3,79 (m, 4H) ; 3,70 - 4,00 (m, 4 H) ; 3,24 (s, 6H) ; 3,14 (m, 2H) ; 2,30 - 2,65 (m, 2H); 1,97 (m, 2H).
³¹P {¹H} RMN (CD₃OD + D₂O, 161 MHz) : δ 16,41 ppm.
SM-ESI (méthanol + 2% TfA) 476,1 ([M²⁺], 100), 951,2 ([M⁺], 55).

Analyse élémentaire pour C₃₈H₄₁N₈Na₇O₁₇P₂, 2H₂O :

| | **C** | **H** | **N** |
|---|---|---|---|
| **Calculé** | **40,01** | **3,98** | **9,82** |
| **Trouvé** | **39,79** | **3,68** | **9,67** |

### EXEMPLE 2 :

### Synthèse du composé de formule 13

Un composé 13 a été synthétisé selon le schéma réactionnel suivant :

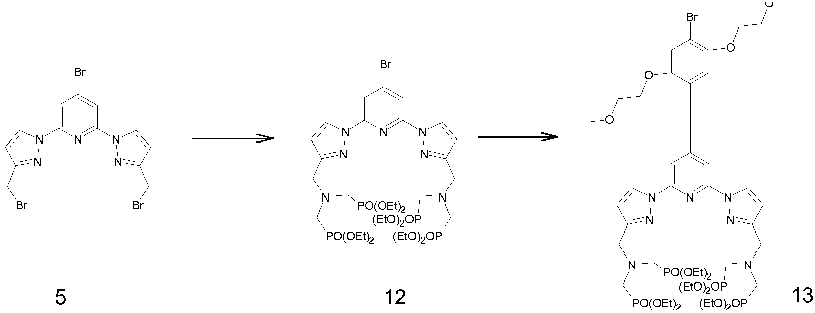

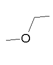

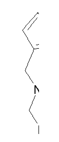

### 1) Première étape : Synthèse du composé 12

A une solution de 256,4 mg du composé **5** tel qu'utilisé ci-dessus à la première étape de l'exemple 1 (0,539 mmol) dans 10 ml d'acétonitrile anhydre, on a ajouté 512,7 mg de dérivé amino-di(méthylènediéthylphosphite) (1,616 mmol) et 297,8 mg de K₂CO₃ anhydre (2,155 mmol). La suspension résultante a été chauffée à 60°C pendant 12 h sous argon. Le composé a été purifié par chromatographie sur gel de silice en utilisant un mélange de solvants (de 0/10 à 4/6 (v/v) MeOH/CH₂Cl₂) et a donné le composé **12** attendu sous forme d'un solide blanc avec un rendement de 65%.
¹H RMN (300 MHz, CDCl₃) δ (ppm) : 8,36 (d, J = 2,6 Hz, 2 H) ; 7,87 (s, 2 H) ; 6,48 (d, J = 2,6 Hz, 2 H) ; 4,01 - 4,14 (m, 20 H) ; 3,16 (s, 4 H) ; 3,12 (s, 4 H) ; 1,25 (t, J = 7,0 Hz, 24 H).
³¹P {¹H} RMN (CDCl₃, 161 MHz) : δ 24,76 ppm.
SM-ESI 949,1 (100), 947,1 (90).

Analyse élémentaire pour C₃₃H₅₈N₇O₁₂P₄Br :

| | **C** | **H** | **N** |
|---|---|---|---|
| **Calculé** | **41,78** | **6,16** | **10,34** |
| **Trouvé** | **41,34** | **6,00** | **10,09** |

### 2) Deuxième étape : Synthèse du composé 13

A une solution dégazée de 100 mg du composé **12** obtenu ci-dessus à l'étape précédente (0,105 mmol) dans un mélange benzène/triéthylamine (5 ml/1 ml) a été additionné 41,6 mg de composé **4** (0,126 mmol) et 10 mg de [Pd(PPh₃)₄]. La solution a été chauffée à 60°C durant la nuit et le composé **13** attendu a été isolé par chromatographie sur silice. Le composé pur a été obtenu avec un rendement de 83% sous forme d'un solide blanc cristallin.
¹H RMN (300 MHz, CDCl₃) δ (ppm) : 8,42 (d, J = 2,7 Hz, 2H) ; 7,96 (s, 2H) ; 6,80 (s, 1H) ; 6,74 (s, 1H) ; 6,53 (d, J = 2,7 Hz, 2H) ; 4,13 (m, 4H) ; 4,01 - 4,14 (m, 20H) ; 3,80 (m, 4H) ; 3,27 (s, 6H) ; 3,17 (s, 4H) ; 3,12 (s, 4H) ; 1.27 (t, J = 7,0 Hz, 24H).
³¹P {¹H} RMN (CDCl₃, 161 MHz) : δ 24,87 ppm.
SM-ESI 1197,2 (100), 1195,2 (90).

Analyse élémentaire pour C₄₇H₇₄N₇O₁₆P₄Br :

| | **C** | **H** | **N** |
|---|---|---|---|
| **Calculé** | **47,16** | **6,23** | **8,19** |
| **Trouvé** | **46,87** | **6,02** | **7,76** |

### EXEMPLE 3 :

### Synthèse du composé de formule (I-3)

Un composé de formule (I-3) a été synthétisé selon le schéma réactionnel suivant :

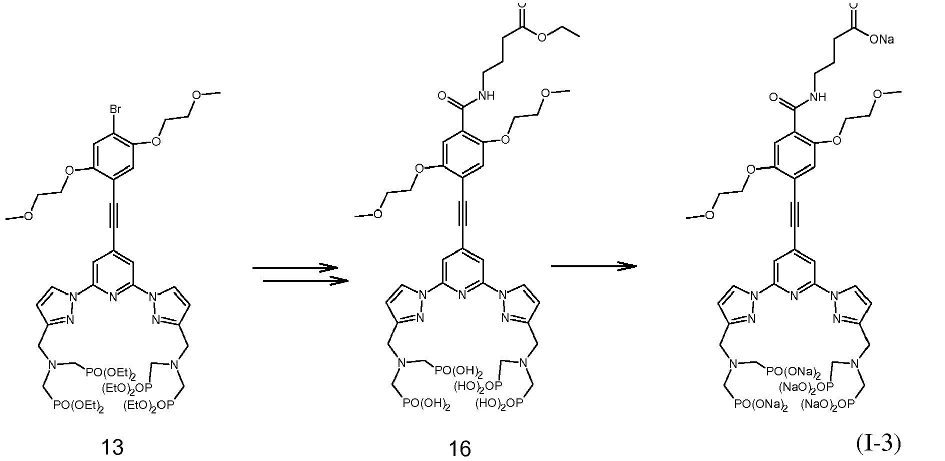

### 1) Première étape : Synthèse du composé 16

A une solution de 115,0 mg du composé **13** obtenu ci-dessus à l'étape 2) de l'exemple 2 (0,096 mmol) dans un mélange de 8 ml de toluène et de 8 ml de triéthylamine, on a ajouté 51,7 mg de chlorhydrate de 4-éthylaminobutyrate (0,308 mmol) et 9,8 mg de Pd(PPh₃)₂Cl₂ (0,014 mmol). La solution résultante a été chauffée à 70°C durant 12 h sous un flux continu de CO. A la fin de la réaction, le solvant a été évaporé, le résidu a été traité à l'eau et extrait avec du dichlorométhane sec. Le solide blanc a été séché et dissous dans de l'acétonitrile anhydre (10 ml). On y a ajouté 740 mg de bromure de triméthylsilyle fraîchement distillé (4,84 mmol) et 519 mg de 2,6-lutidine anhydre (4,843 mmol). La solution résultante a été agitée à température ambiante durant 48 h sous argon. La solution a été évaporée à sec, le résidu a été dissous dans de l'eau et le pH de la solution a été ajusté à 7 avec une solution de soude (0,1M à 0,01M). Le composé **16** attendu a été obtenu avec un rendement chimique de 58% pour les deux étapes de synthèse.
¹H RMN (200 MHz, D₂O) δ (ppm) : 8,58 (s, 2 H) ; 7,46 (s, 2 H) ; 6,86 (s, 2 H) ; 6,80 (s, 1H) ; 6,76 (s, 1H) ; 4,15 (m, 4H) ; 4,09 (m, 4H) ; 3,76 (t, 4H) ; 3,46 - 3,78 (m, 8H) ; 3,26 (s, 6H) ; 2,48 - 2,70 (m, 3H) ; 1,62 - 2,00 (m, 4 H) ; 1,28 - 1,48 (m, 3 H); 3,65 (s, 4 H); 3,26 (s, 2 H); 3,20 (s, 2 H); 2,43 (t, J = 7,0 Hz, 2 H).
³¹P {¹H} RMN (CD₃OD+D₂O, 161 MHz) : δ 8,78 ppm.
SM-ESI 1050,1 (100).

Analyse élémentaire pour C₃₈H₅₄N₈O₁₉P₄ :

| | **C** | **H** | **N** |
|---|---|---|---|
| **Calculé** | **43,44** | **5,18** | **10,66** |
| **Trouvé** | **43,09** | **4,76** | **10,23** |

### 2) Deuxième étape : Synthèse du composé (I-3)

A une solution de 94,3 mg du composé **16** obtenu ci-dessus à l'étape précédente (0,090 mmol) dans 4 ml d'eau, on a ajouté 31,0 mg de soude (0,775 mmol). La solution a été agitée sous argon à température ambiante durant 24 heures. Après évaporation du solvant le résidu a été dissous dans du méthanol et le composé a été précipité avec de l'éther. On a récupéré 95 % du composé **(I-3)** attendu analytiquement pur.
¹H RMN (200 MHz, D₂O) δ (ppm) : 8,62 (s, 2 H) ; 7,53 (s, 2 H) ; 6,83 (s, 2 H) ; 6,78 (s, 1H) ; 6,77 (s, 1H) ; 4,20 (t, 4 H) ; 4,11 (m, 4H) ; 3,74 (m, 4H) ; 3,32 (s, 6H) ; 2,52 - 2,83 (m, 4 H) ; 3,71 (s, 4 H) ; 3,31 (s, 2 H) ; 3,19 (s, 2 H) ; 1,41 - 1,61 (m, 3 H).
³¹P {¹H} RMN (D₂O, 161 MHz) : δ 15,97 ppm.
SM-ESI (méthanol + 2% TfA) 509,5 ([M2+], 100), 1018,1 ([M+], 35).

Analyse élémentaire pour C₃₆H₄₁N₈Na₉O₁₉P₄, 2H₂O :

| | **C** | **H** | **N** |
|---|---|---|---|
| **Calculé** | **34,41** | **3,61** | **8,92** |
| **Trouvé** | **34,17** | **3,21** | **8,78** |

### EXEMPLE 4 :

### Synthèse du composé de formule (I-5)

Un composé de formule **(I-5)** a été synthétisé selon le schéma réactionnel suivant :

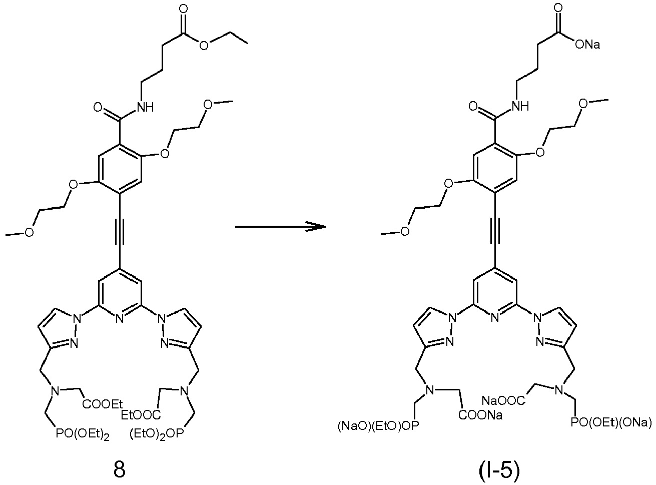

A une solution du composé **8** (50 mg, 0,044 mmol) obtenu ci-dessus à la troisième étape de préparation du composé de formule **(I-1),** dans de l'eau (10 ml), on a ajouté 5 ml d'une solution aqueuse de soude à 1M. La solution a été agitée à température ambiante durant 12 heures. L'addition lente de diéthyléther a provoqué la précipitation du composé **(I-5)** désiré. Ce précipité a été centrifugé à 3000 tours par minute, lavé avec de l'éther. Le composé résultant a été recristallisé 2 fois par diffusion d'éther dans une solution concentrée de méthanol. On a obtenu le composé de formule **(I-5)** attendu avec un rendement de 83%.
¹H RMN (300 MHz, D₂O) δ (ppm) : 8,62 (s, 2H) 8,03 (d, J = 8,0 Hz, 2H) ; 6,78 (s, 2H) ; 6,81 (s, 1H) ; 6,72 (s, 1H) ; 4,83 (s, 4H) ; 4,34 (s, 4H) ; 4,14 (m, 4H) ; 3,82 (m, 4H) ; 3,62 (m, 4H), 3,62 - 4,04 (m, 4H) ; 3,18 (s, 6H) ; 3,15 (m, 2H) ; 2,32 - 2,63 (m, 2H) ; 1,92 (m, 2 H) ; 1,12 (m, 6H).
³¹P {¹H} RMN (CD₃OD + D₂O, 161 MHz) : δ 28,21 ppm.
SM-ESI (méthanol + 2% TfA) 1007,2 ([M⁺], 100).

Analyse élémentaire pour C₄₂H₅₁N₈Na₅O₁₇P₂, 2H₂O :

| | **C** | **H** | **N** |
|---|---|---|---|
| **Calculé** | **43,76** | **4,81** | **9,72** |
| **Trouvé** | **43,64** | **4,49** | **9,53** |

### EXEMPLE 5 :

### Synthèse du composé de formule (I-7)

Un composé de formule **(I-7)** a été synthétisé selon le schéma réactionnel suivant :

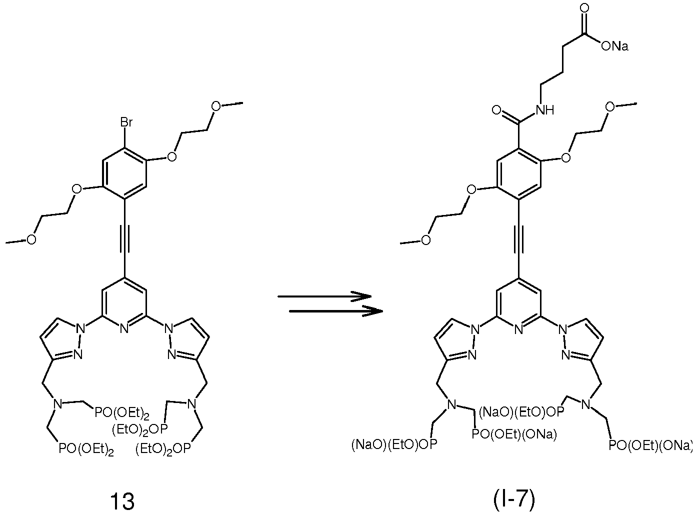

A une solution de 100 mg (0,083 mmol) du composé **13** obtenu ci-dessus à la deuxième étape de l'exemple 2 dans un mélange de 5 ml de toluène et de 1 ml de triéthylamine, on a ajouté 52 mg de chlorhydrate de 4-éthylaminobutyrate (0,308 mmol) et 10 mg de Pd(PPh₃)₂Cl₂ (0,014 mmol). La solution résultante a été chauffée à 70°C durant 12 h sous un flux continu de CO. A la fin de la réaction, le solvant a été évaporé, le résidu a été traité à l'eau et extrait avec du dichlorométhane sec. Le solide blanc a été séché et dissous dans de l'eau (10 ml), on a ajouté 20 ml d'une solution aqueuse de soude (1N). La solution a été agitée à température ambiante durant 12 heures. L'addition lente de diéthyléther a provoqué la précipitation du composé **(I-7)** désiré. Ce précipité a été centrifugé à 3000 tours par minute, lavé avec de l'éther. Le composé résultant a été recristallisé 2 fois par diffusion d'éther dans une solution concentrée de méthanol. On a obtenu le composé de formule **(I-7)** attendu avec un rendement de 52%.
¹H RMN (200 MHz, D₂O δ (ppm) : 8,65 (s, 2H) ; 7,64 (s, 2H) ; 6,86 (s, 2H) ; 6,79 (s, 1H) ; 6,80 (s, 1H) ; 4,25 (t, 4H) ; 4,15 (m, 4H) ; 3,86 (m, 4H) ; 3,52 (m, 8H), 3,32 (s, 6H) ; 2,43 - 2,86 (m, 4H) ; 3,75 (s, 4H) ; 3,35 (s, 2H) ; 3,23 (s, 2H) ; 1,42 - 1,64 (m, 3H), 1,14 (m, 12H).
³¹P {¹H} RMN (D₂O, 161 MHz) : δ 28,27 ppm.
SM-ESI (méthanol + 2% TfA) 1035,2 ([M⁺], 100).

Analyse élémentaire pour C₄₄H₆₁N₈Na₅O₁₉P₄, 2H₂O :

| | **C** | **H** | **N** |
|---|---|---|---|
| **Calculé** | **41,26** | **5,11** | **8,75** |
| **Trouvé** | **41,01** | **4,86** | **8,46** |

## Revendications

1. Composé de formule (I) suivante : 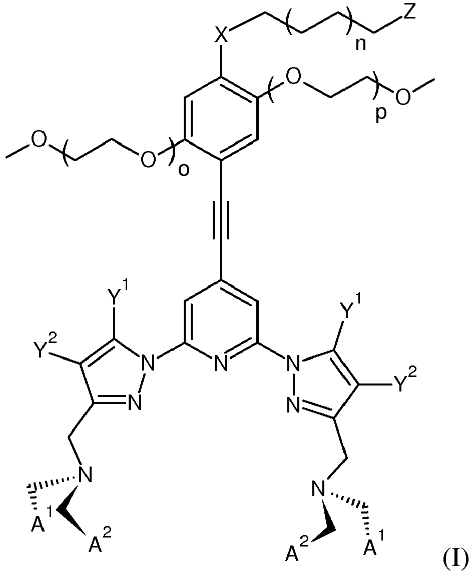 dans laquelle :
- n, o, p, indépendamment les uns des autres, sont des nombres entiers variant de 0 à 4 ;
- A¹ représente une fonction -COOR¹ dans laquelle R¹ est un atome d'hydrogène ou un cation alcalin ; ou un groupement A² ;
- A² représente un groupement -P(O)(OCH₂CH₃)(OR²) ou -P(O)(OR²)₂ dans lesquels R² représente un atome d'hydrogène ou cation alcalin ;
- chacun des Y¹, Y² représente un atome d'hydrogène ou bien Y¹ et Y² forment ensemble un biradical formant un ou plusieurs cycles aromatiques ou hétéroaromatique avec les deux atomes de carbone qui les portent, lesdits cycles aromatiques portant éventuellement un ou plusieurs substituants choisis parmi un atome d'hydrogène, un groupement amino et un groupement thiol ;
- X représente un segment de liaison constitué par une fonction amide -C(O)-NH- ;
- Z est:
* NH₂, un halogène, un phosphate,
* un groupe COOR³ dans lequel R³ est H, un cation alcalin, un groupe ammonium quaternaire N(R⁴)₄⁺ dans lequel R⁴ est H ou une chaîne alkyle linéaire ayant de préférence de 1 à 4 atomes de carbone, un groupe succinimide -N-(CO-CH₂-CH₂-CO)- ou un groupe pentafluorophényle -C₆F₅ ;
* une fonction biotine (-CO-(CH₂)₄-C₅H₇N₂OS), un groupement polyhétéroaromatique ou un éther couronne,
* un cluster organique ou inorganique silylé ;
* un support macroscopique.

2. Composé selon la revendication 1, **caractérisé en ce que** R¹ et R² sont choisis parmi K⁺, Na⁺ et Li⁺.

3. Composé selon la revendication 1 ou 2, **caractérisé en ce que** les groupes Y¹ et Y² font partie d'un cycle aromatique ou hétéroaromatique.

4. Composé selon l'une quelconque des revendications précédentes, **caractérisé en ce qu'**il répond à l'une des formules (I-1), (I-3), (I-5) ou (I-7) suivantes :

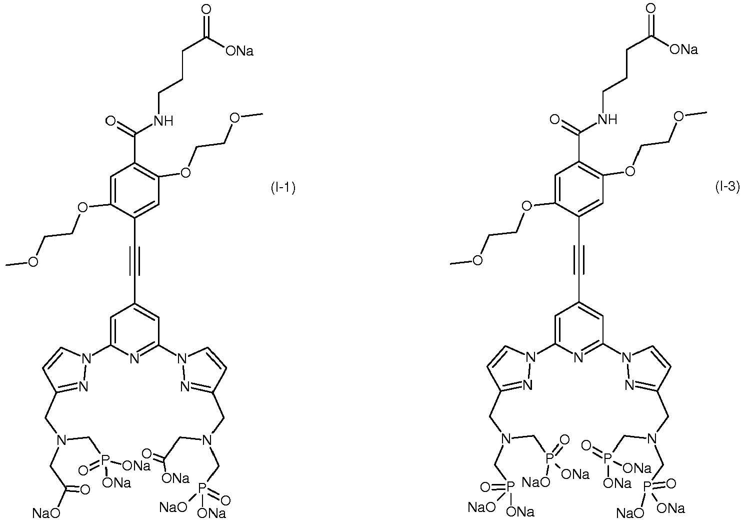

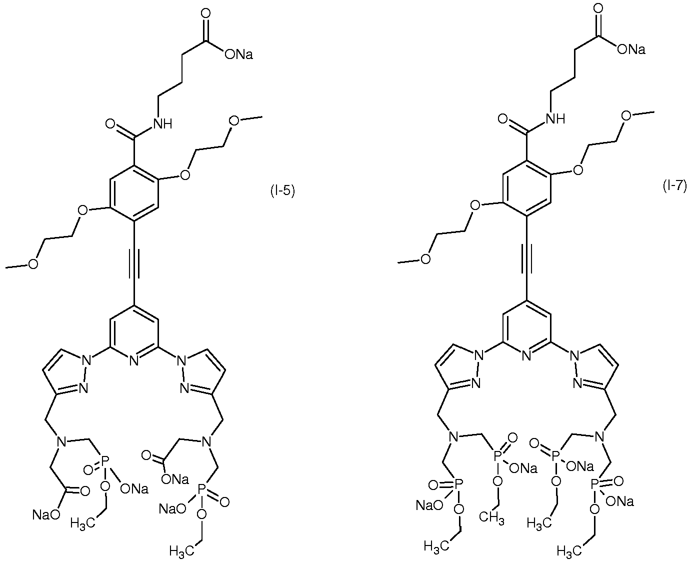

5. Procédé de préparation d'un composé de formule (I) tel que défini à l'une quelconque des revendications précédentes, **caractérisé en ce qu'**il comprend les étapes suivantes :
1) une première étape de substitution nucléophile des atomes de brome d'un composé de formules (V) ou (V') suivantes : 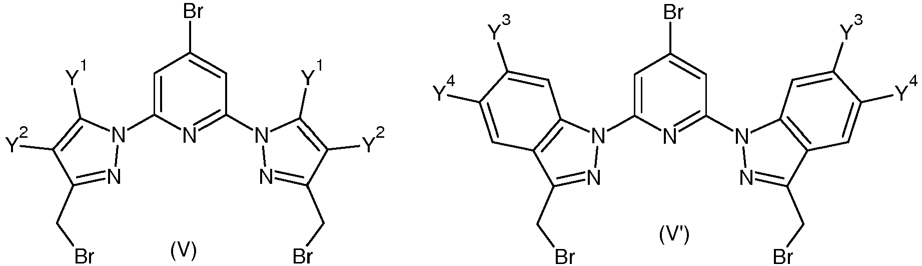 dans lesquelles
- les groupements Y¹, Y², ont la même signification que celle indiquée à la revendication 1 pour les composés de formule (I) ;
- les groupements Y³ et Y⁴, identiques ou différents, sont choisis parmi un atome d'hydrogène, un groupement amino et un groupement thiol ;
ladite substitution nucléophile étant réalisée dans un solvant anhydre et à une température comprise entre environ 60°C et 80°C , en présence d'une base minérale et d'un composé choisi parmi les composés de formule (III) suivante [NH(CH₂COOR'¹)(CH₂P(O)(OR'²)₂] et les composés de formule (IV) suivante [NH{CH₂P(O)(OR'²)₂}₂] dans lesquelles R'¹ et R'², indépendamment l'un de l'autre, représentent un radical alkyle linéaire ou ramifié en C₁-C₄, pour obtenir un composé de formule (VI), respectivement de formule (VI'), suivantes : 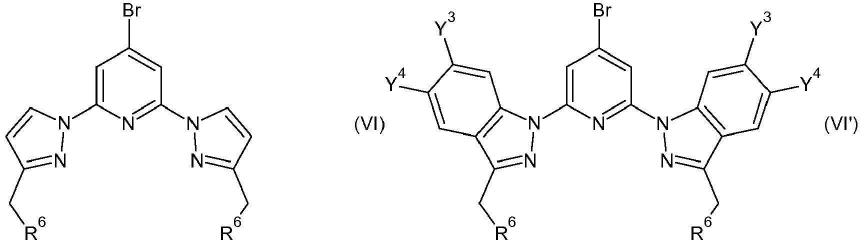 dans lesquelles R⁶ est choisi parmi les groupements de formule R⁶-a ou R⁶-b suivants : 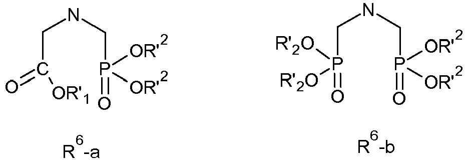 dans lesquels :
- R'¹ représente un radical alkyle linéaire ou ramifié en C₁-C₄ ;
- R'² représente un radical C₁-C₄ ;
ii) une deuxième étape de préparation d'un composé 4 de formule suivante : 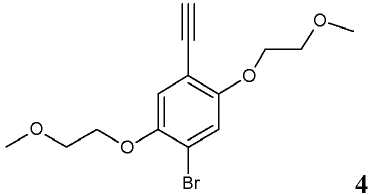 selon un procédé comprenant les sous-étapes suivantes :
ii-a) la mise en réaction du paradiphénol avec du tosylate de 2-méthoxyéthanol, dans un solvant anhydre en présence d'une base minérale, à une température d'environ 80°C pendant 12 heures environ pour obtenir un composé **1** de formule suivante : 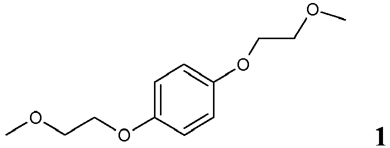
ii-b) une étape de bromation du composé **1** obtenu à l'étape précédente par du brome (Br₂), dans un solvant au reflux pendant environ 12 heures pour obtenir un composé **2** de formule suivante : 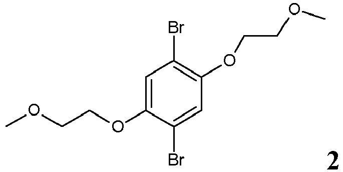
ii-c) une étape de substitution d'un des deux atomes de brome du composé **2** obtenu à l'étape précédente, dans un solvant, en présence de di-isopropylamine, d'un réactif (CH₃)₂C(OH)C≡CH, d'un catalyseur au palladium, et de CuI, pour obtenir un composé **3** de formule suivante : 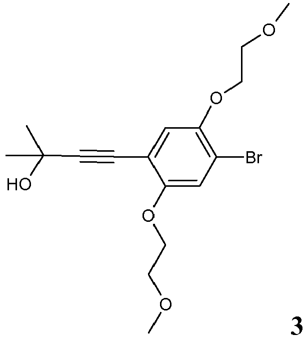
ii-d) une étape d'hydrolyse alcaline du composé **3** obtenu ci-dessus à l'étape précédente par une base forte anhydre, dans un solvant organique à une température d'environ 130°C pendant 12 heures environ, pour obtenir ledit composé **4** ;
iii) une troisième étape de couplage croisé entre le composé **4** obtenu ci-dessus à l'étape ii) et le composé de formule (VI), respectivement (VI') obtenu ci-dessus à l'étape i), dans un solvant organique en présence d'un catalyseur au palladium et de triphénylphosphine, pour obtenir un composé de formule (VII), respectivement (VII'), suivantes : 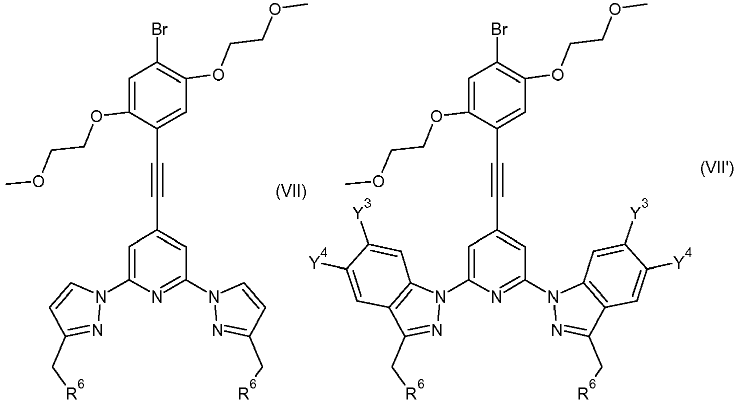 dans lesquelles R⁶ a la même signification que dans les composés de formule (VI), respectivement (VI'), ci-dessus ;
iv) une quatrième étape au cours de laquelle le brome du composé de formule (VII), respectivement (VII') est remplacé par un groupe de formule X-CH₂-(CH₂CH₂)ₙ-CH₂-Z dans lequel n, X et Z ont la même signification que celle indiquée à la revendication 1 relativement aux composés de formule (I), par réaction avec un réactif approprié, en présence d'un catalyseur organométallique, dans un solvant organique ;
v) une cinquième étape au cours de laquelle les groupes R'¹ et R'² sont respectivement remplacés par des groupes R¹ et R², lesdits groupements étant H ou un cation alcalin (pour R¹) ou un cation alcalin (pour R²).

6. Procédé selon la revendication 5, **caractérisé en ce que** lors de la cinquième étape, le remplacement des groupes R'¹ et R'² du type alkyle linéaire par un cation K⁺, Na⁺ ou Li⁺ est effectué avec KOH, NaOH ou LiOH respectivement dans un solvant polaire, à une température entre 20 et 100°C, ou bien en utilisant du bromure de triméthylsilyle dans un solvant à température ambiante suivi d'une hydrolyse basique.

7. Procédé selon la revendication 5, **caractérisé en ce que** lors de la cinquième étape, le remplacement par H de groupes R'¹ ou R'² du type alkyle ramifié est effectué par réaction avec l'acide trifluoroacétique dans un solvant organique aprotique.

8. Complexe de lanthanide ou d'un métal de transition, **caractérisé en ce qu'**il comprend un ion lanthanide ou d'un métal de transition complexé par un ligand de formule (I) tel que défini à l'une quelconque des revendications 1 à 4.

9. Complexe selon la revendication 8, **caractérisé en ce que** l'ion lanthanide est choisi parmi les ions Gd³⁺, Lu³⁺, Eu³⁺, Tb³⁺, Dy³⁺, Sm³⁺, Er³⁺, Yb³⁺, Pr³⁺ et Nd³⁺.

10. Complexe selon la revendication 8, **caractérisé en ce que** l'ion d'un métal de transition est choisi parmi les ions Cu(II), Co(II), Mn(II ou IV), Ni(II), Fe(III), Pd(II) et Pt(II).

11. Utilisation d'un complexe tel que défini à l'une quelconque des revendications 8 à 10, dans lequel le groupe Z du ligand du complexe est un groupe -COOH, un groupe COONa, un ester activé de type ester de *N*-hydroxysuccinimide ou ester de pentafluorophénol, un Br, un I, ou une amine, pour le marquage de composés qui portent un groupe amine, alcool, thiol, acide carboxylique ou ester activé.

12. Utilisation d'un complexe tel que défini à l'une quelconque des revendications 8 à 10, comme marqueur luminescent pour l'absorption à deux photons.

13. Utilisation d'un complexe tel que défini à l'une quelconque des revendications 8 à 10, dans lesquels le groupe terminal Z du ligand est un groupe biotine, pour la reconnaissance de l'avidine, de la streptavidine et de la neuravidine.

## Patentansprüche

1. Verbindung der folgenden Formel (I): 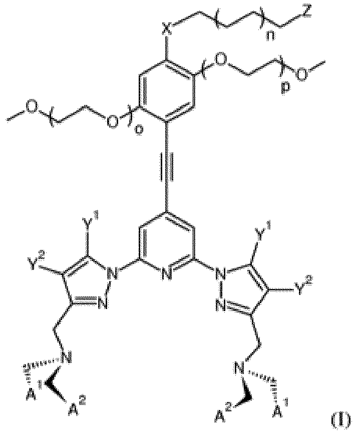 in welcher:
- n, o, p unabhängig voneinander Ganzzahlen zwischen 0 und 4 sind,
- A¹ eine Funktion -COO¹ darstellt, in welcher R¹ ein Wasserstoffatom oder ein alkalisches Kation ist, oder eine Gruppe A²,
- A² eine Gruppe -P(O)(OCH₂CH₃)(OR²) oder -P(O)(OR²)₂ darstellt, in welchen R² ein Wasserstoffatom oder alkalisches Kation darstellt,
- wobei jeder der Y¹, Y² ein Wasserstoffatom darstellt oder Y¹ und Y² gemeinsam ein Biradikal bilden, das mit den zwei Kohlenstoffatomen, die sie tragen, einen oder mehrere aromatische oder heteroaromatische Cyclen bildet, wobei die aromatischen Cyclen eventuell einen oder mehrere Substituenten tragen, die aus einem Wasserstoffatom, einer Aminogruppe und einer Thiolgruppe ausgewählt sind,
- X ein Verbindungssegment darstellt, das von einer Amidfunktion -C(O)-NH-gebildet ist,
- Z ist:
* NH₂, ein Halogen, ein Phosphat,
* eine Gruppe COOR³, in welcher R³ H, ein alkalisches Kation, eine quaternäre Amoniumgruppe N(R⁴)₄⁺ ist, in welcher R⁴ H oder eine lineare Alkylkette ist, mit vorzugsweise 1 bis 4 Kohlenstoffatomen, eine Succinimidgruppe -N-(CO-CH₂-CH₂-CO)- oder eine Pentafluorphenylgruppe -C₆F₅;
* eine Biotinfunktion (-CO-(CH₂)₄-C₅H₇N₂OS), eine polyheteroaromatische Gruppe oder ein Kronenether,
* ein organischer oder anorganischer silylsierter Cluster,
* ein makroskopischer Support.

2. Verbindung nach Anspruch 1, **dadurch gekennzeichnet, dass** R¹ und R² aus K⁺, Na⁺ und Li⁺ ausgewählt sind.

3. Verbindung nach Anspruch 1 oder 2, **dadurch gekennzeichnet, dass** die Gruppen Y¹ und Y² Teil eines aromatischen oder heteroaromatischen Cyclus sind.

4. Verbindung nach einem der vorangehenden Ansprüche, **dadurch gekennzeichnet, dass** sie einer der folgenden Formeln (I-1 ), (I-3), (I-5) oder (I-7) entspricht:

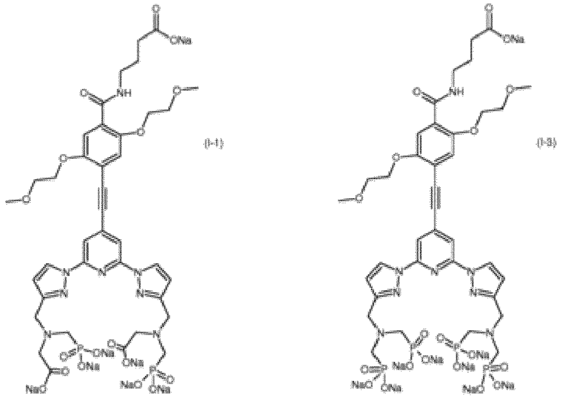

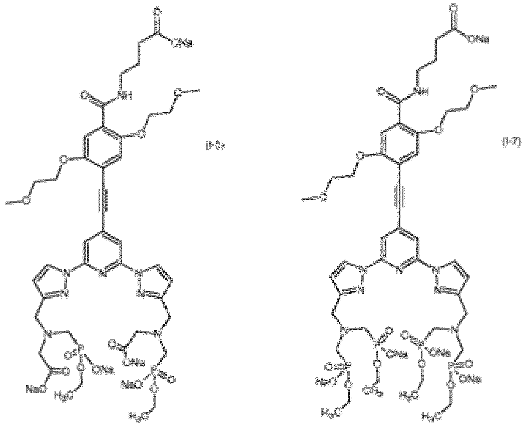

5. Herstellungsverfahren einer Verbindung der Formel (I) nach einem der vorangehenden Ansprüche, **dadurch gekennzeichnet, dass** es die folgenden Schritte umfasst:
1) einen ersten Schritt der nukleophilen Substitution der Bromatome einer Verbindung der folgenden Formeln (V) oder (V'): 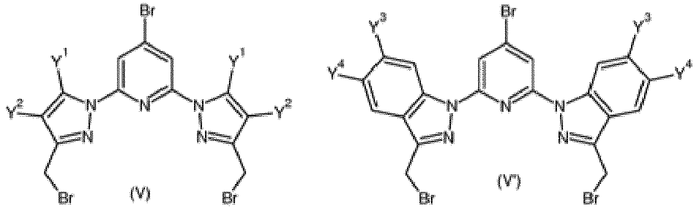 in welchen
- die Gruppen Y¹, Y² dieselbe Bedeutung haben wie die, die in Anspruch 1 für die Verbindungen der Formel (I) angegeben ist,
- die Gruppen Y³ und Y⁴, identisch oder unterschiedlich, aus einem Wasserstoffatom, einer Aminogruppe und einer Thiolgruppe ausgewählt sind,
wobei die nukleophile Substitution in einem wasserfreien Lösungsmittel und bei einer Temperatur von zirka 60 °C bis 80°C durchgeführt wird bei Anwesenheit einer mineralischen Basis und einer Verbindung, ausgewählt aus den Verbindungen der folgenden Formel (III) [NH(CH₂COO'¹)(CH₂P(O)(O'²)₂] und den Verbindungen der folgenden Formel (IV) [NH{CH₂P(O)(OR'²)₂}₂], in welchen R'¹ und R'², unabhängig voneinander, ein lineares oder verzweigtes C₁-C₄-Alkylradikal darstellen, um eine Verbindung der folgenden Formel (VI) beziehungsweise der folgenden Formel (VI') zu erhalten: 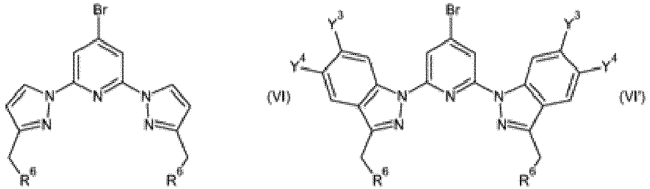 in welchen R⁶ aus den folgenden Gruppen der Formel R⁶-a oder R⁶-b ausgewählt ist: 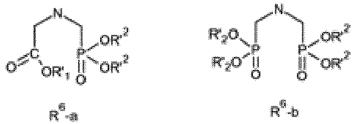 in welchen:
- R'¹ ein lineares oder verzweigtes C₁-C₄-Alkylradikal darstellt,
- R'² ein C₁-C₄-Radikal darstellt,
ii) einen zweiten Schritt der Herstellung einer Verbindung **4** der folgenden Formel: 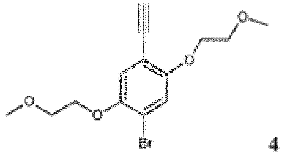 gemäß einem Verfahren, umfassend die folgenden Unterschritte:
ii-a) das Inreaktionversetzen des Paradiphenols mit 2-Methoxyethanoltosylat in einem wasserfreien Lösungsmittel bei Anwesenheit einer mineralischen Basis bei einer Temperatur von zirka 80 °C während zirka 12 Stunden, um eine Verbindung **1** der folgenden Formel zu erhalten: 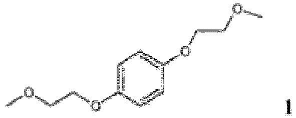
ii-b) einen Bromierungsschritt der Verbindung **1**, erhalten in vorangehendem Schritt, mittels Brom (Br₂), in einem Rückfluss-Lösungsmittel während zirka 12 Stunden, um eine Verbindung **2** der folgenden Formel zu erhalten: 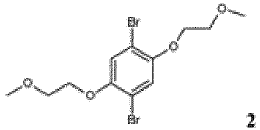
ii-c) einen Substitutionsschritt eines der beiden Bromatome der Verbindung **2**, erhalten in vorangehendem Schritt, in einem Lösungsmittel, bei Anwesenheit von Diisopropylamin, eines Reagens (CH₃)₂C(OH)C≡CH, eines Palladiumkatalysators und von Cul, um eine Verbindung **3** der folgenden Formel zu erhalten: 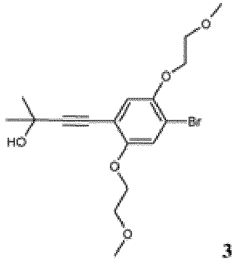
ii-d) einen alkalischen Hydrolyseschritt der obigen Verbindung **3**, erhalten in vorangehendem Schritt, mittels einer starken wasserfreien Base, in einem organischen Lösungsmittel bei einer Temperatur von zirka 130 °C während zirka 12 Stunden, um die Verbindung **4** zu erhalten,
iii) einen dritte Kreuzkopplungsschritt zwischen der Verbindung **4**, erhalten in obigem Schritt ii), und der Verbindung der Formel (VI) beziehungsweise (VI'), erhalten in obigem Schritt i), in einem organischen Lösungsmittel bei Anwesenheit eines Palladiumkatalysators und von Triphenylphosphin, um eine Verbindung der folgenden Formel (VII) beziehungsweise der folgende Formel (VII') zu erhalten: 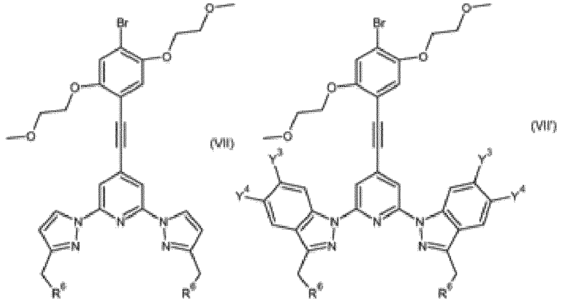 in welchen R⁶ dieselbe Bedeutung wie in den Verbindungen der obigen Formeln (VI) beziehungsweise (VI') hat,
iv) einen vierten Schritt, bei dem das Brom der Verbindung der Formel (VII) beziehungsweise (VII') von einer Gruppe der Formel X-CH₂-(CH₂CH₂)ₙ-CH₂-Z ersetzt wird, in welcher n, X und Z dieselbe Bedeutung haben wie die, die in Anspruch 1 in Bezug auf Verbindungen der Formel (I) angegeben ist, durch Reaktion mit einem geeigneten Reagens, bei Anwesenheit eines organometallischen Katalysators, in einem organischen Lösungsmittel,
v) einen fünften Schritt, bei dem die Gruppen R'¹ und R'² jeweils von den Gruppen R¹ und R² ersetzt werden, wobei die Gruppen H oder ein alkalisches Kation (für R¹) oder ein alkalisches Kation (für R²) sind.

6. Verfahren nach Anspruch 5, **dadurch gekennzeichnet, dass** beim fünften Schritt der Ersatz der Gruppen R'¹ und R'² des Typs lineares Alkyl durch ein Kation K⁺, Na⁺ oder Li⁺ mit KOH, NaOH oder LiOH jeweils in einem polaren Lösungsmittel bei einer Temperatur zwischen 20 und 100 °C oder bei Verwendung von Trimethylsilylbromid in einem Lösungsmittel bei Raumtemperatur, gefolgt von einer basischen Hydrolyse, durchgeführt wird.

7. Verfahren nach Anspruch 5, **dadurch gekennzeichnet, dass** beim fünften Schritt der Ersatz durch H von Gruppen R'¹ oder R'² des Typs verzweigtes Alkyl durch Reaktion mit Trifluoressigsäure in einem aprotischen Lösungsmittel durchgeführt wird.

8. Lanthanidkomplex oder eines Übergangsmetalls, **dadurch gekennzeichnet, dass** er ein Lanthanidion oder eines Übergangsmetalls umfasst, komplexiert mittels eines Liganden der Formel (I), wie nach einem der Ansprüche 1 bis 4 definiert.

9. Komplex nach Anspruch 8, **dadurch gekennzeichnet, dass** das Lanthanidion aus den Ionen Gd³⁺, Lu³⁺, Eu³⁺, Tb³⁺, Dy³⁺, Sm³⁺, Er³⁺, Yb³⁺, Pr³⁺ und Nd³⁺ ausgewählt ist.

10. Komplex nach Anspruch 8, **dadurch gekennzeichnet, dass** das Ion eines Übergangsmetalls aus den Ionen Cu(II), Co(II), Mn(II oder IV), Ni(II), Fe(III), Pd(II) und Pt(II) ausgewählt ist.

11. Verwendung eines Komplexes nach einem der Ansprüche 8 bis 10, in welchem die Gruppe Z des Liganden des Komplexes eine Gruppe -COOH, eine Gruppe COONa, ein aktivierter Ester vom Typ N-Hydroxysuccinimidester oder Pentafluorphenolester, ein Br, ein I, oder ein Amin ist für die Markierung von Verbindungen, die eine Amin-, Alkohol-, Thiol-, Carboxylsäure- oder aktivierte Estergruppe tragen.

12. Verwendung eines Komplexes nach einem der Ansprüche 8 bis 10 als lumineszierender Marker für die Absorption mit zwei Photonen.

13. Verwendung eines Komplexes nach einem der Ansprüche 8 bis 10, in welchen die terminale Gruppe Z des Liganden eine Biotingruppe ist für die Erkennung von Avidin, Streptavidin und Neuravidin.

## Claims

1. A compound of following formula (1): 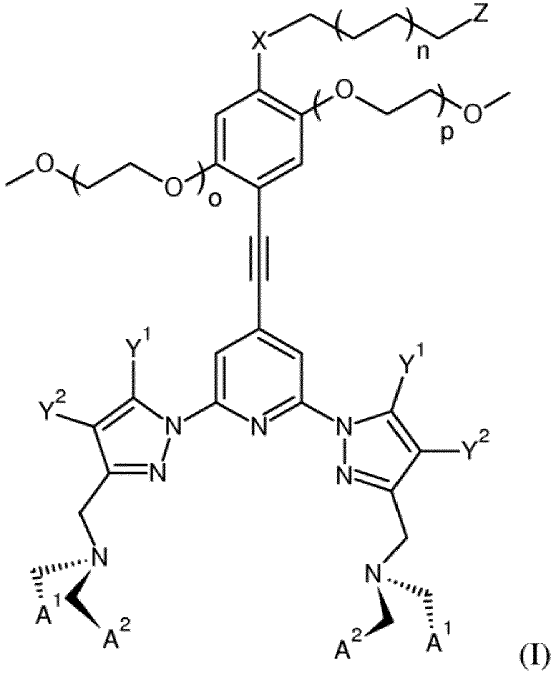 where:
- n, o, p, each independently are integers varying from 0 to 4;
- A¹ is a -COOR¹ function where R¹ is a hydrogen atom or alkaline cation; or an A² group;
- A² is a -P(O)(OCH₂CH₃)(OR²) or -P(O)(OR²)₂ group where R² is a hydrogen atom or alkaline cation;
- each of Y¹, Y² is a hydrogen atom or else Y¹ and Y² together form a biradical forming one or more aromatic rings or heteroaromatic with their two carrier carbon atoms, said aromatic rings optionally carrying one or more substituents selected from among a hydrogen atom, an amino group and thiol group;
- X is a linking segment formed by an amide function -C(O)-NH-;
- Z is:
* NH₂, a halogen, a phosphate;
* a COOR³ group where R³ is H, an alkaline cation, N(R⁴)₄⁺ quaternary ammonium group where R⁴ is H or a straight alkyl chain preferably having 1 to 4 carbon atoms, a -N-(CO-CH₂-CH₂-CO)- succinimide group or -C₆F₅ pentafluorophenyl group;
* a biotin function (CO-(CH₂)₄-C₅H₇N₂OS), polyheteroaromatic group or crown ether;
* a silylated organic or inorganic cluster;
* a macroscopic substrate.

2. The compound according to claim 1, **characterized in that** R¹ and R² are selected from among K⁺, Na⁺ and Li⁺.

3. The composition according to claim 1 or 2, **characterized in that** the groups Y¹ and Y² belong to an aromatic or heteroaromatic ring.

4. The compound according to any of the preceding claims, **characterized in that** it meets one of following formulas (I.1), (1.3), (I-5) or (I-7):

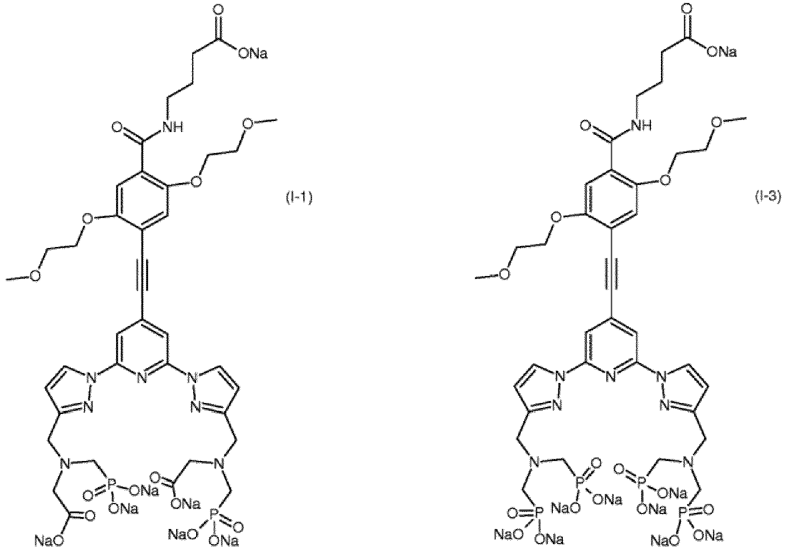

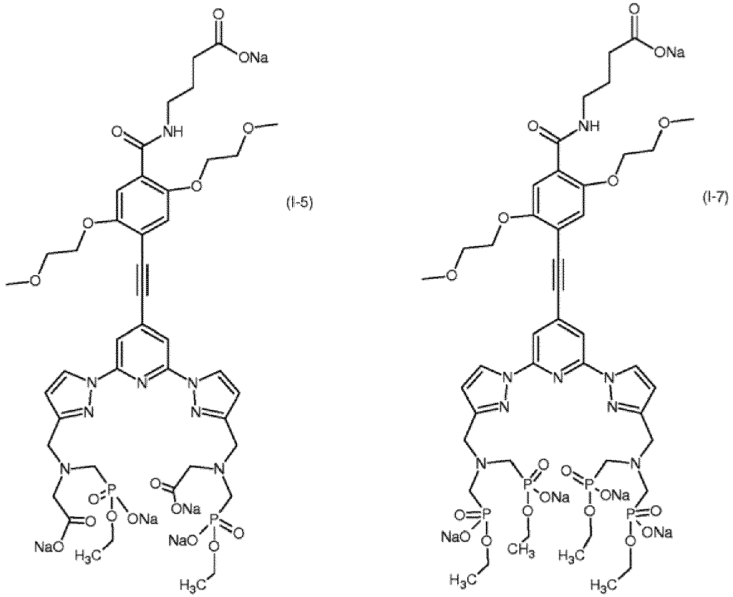

5. A method to prepare a formula (I) compound such as defined in any of the preceding claims, **characterized in that** it comprises the following steps:
1) a first step for nucleophilic substitution of the bromine atoms of a compound of following formulas (V) or (V'): 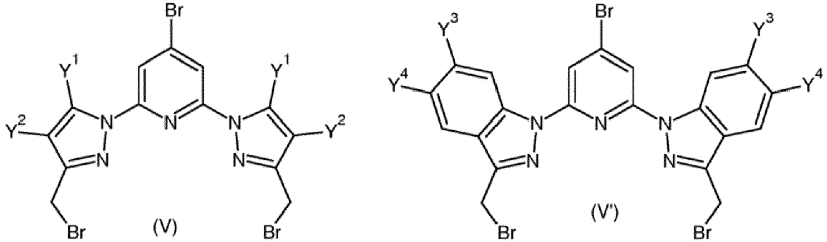 where:
- the groups Y¹, Y² have the same meaning as indicated in claim 1 for the compounds of formula (I);
- the groups Y³ and Y⁴, the same or different, are selected from among a hydrogen atom, an amino group and thiol group;
said nucleophilic substitution being conducted in an anhydrous solvent and at a temperature of between about 60°C and 80°C in the presence of a mineral base and of a compound selected from among the compounds of following formula (III) [NH(CH₂COOR'¹)(CH₂P(O)(OR'2)₂] and the compounds of following formula (IV) [NH{CH₂P(O)(OR'²)₂}₂] where R'¹ and R'² are each independently a C₁-C₄ straight-chain or branched alkyl radical, to obtain a compound of following formula (VI) and formula (VI') respectively: 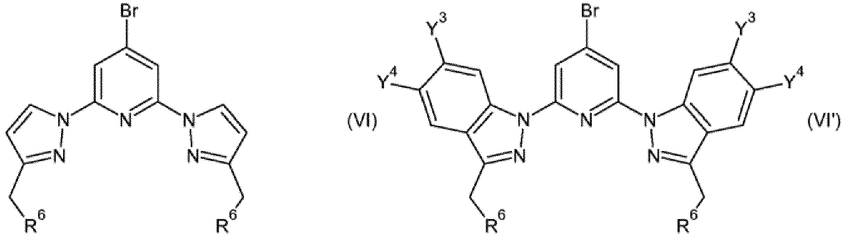 where R⁶ is selected from among the groups of following formulas R⁶-a or R⁶-b 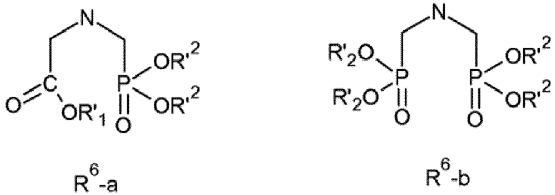 where:
- R'1 is a C₁-C₄ straight-chain or branched alkyl radical;
- R'2 is a C₁ - C₄ radical;
ii) a second step to prepare compound **4** of following formula: 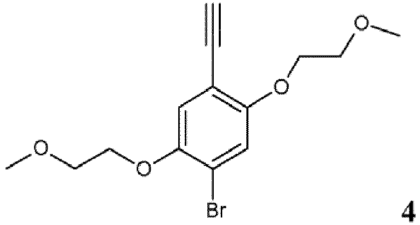 with a method comprising the following sub-steps:
ii-a) reacting paradiphenol with 2-methoxyethanol tosylate in an anhydrous solvent in the presence of a mineral base, at a temperature of about 80°C for about 12 hours to obtain compound **1** of following formula: 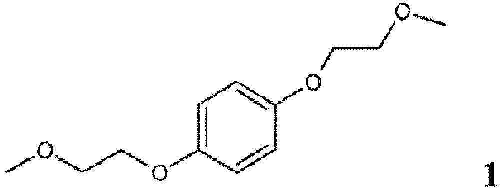
ii-b) a bromination step of compound **1**, obtained at the preceding step. with bromine (Br₂), in a solvent under reflux for about 12 hours to obtain compound **2** of following formula: 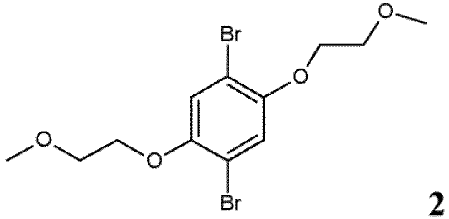
ii-c) a substitution step of one of the two bromine atoms of compound **2** obtained at the preceding step, in a solvent in the presence of di-isopropylamine, a reagent (CH₃)₂C(OH)C≡CH, a palladium catalyst and Cul, to obtain compound **3** of following formula: 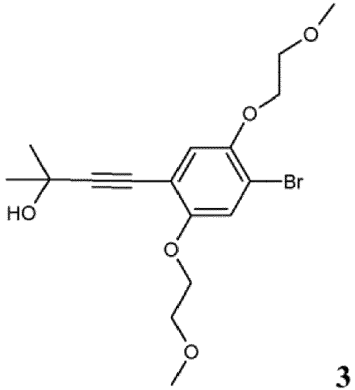
ii-d) an alkaline hydrolysis step of compound **3** obtained at the preceding step with a strong anhydrous base, in an organic solvent, at a temperature of about 130°C for about 12 hours, to obtain said compound **4**;
iii) a third cross-coupling step between compound **4** obtained above at step ii) and the compound of formula (VI) and (VI') respectively obtained above at step i), in an organic solvent in the presence of a palladium catalyst and triphenylphosphine, to obtain a compound of following formula (VII), and (VII') respectively: 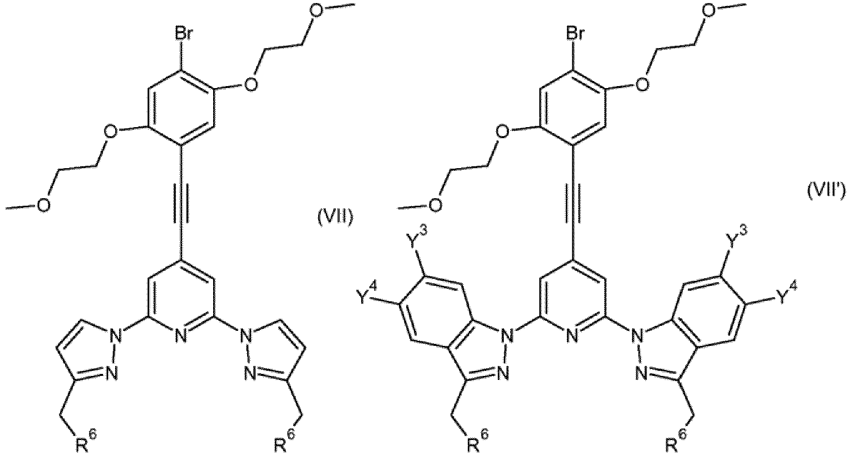 where R⁶ has the same meaning as in the above compounds of formula (VI) and (VI') respectively;
iv) a fourth step at which the bromine of the compound of formula (VII) and (VII') respectively is replaced by a group of formula X-CH₂-(CH₂CH₂)ₙ-CH₂-Z where n, X and Z have the same meaning as indicated in claim 1 regarding the compounds of formula (I), by reaction with a suitable reagent in the presence of an organometallic catalyst in an organic solvent;
v) a fifth step at which the groups R'¹ and R'² are respectively replaced by R¹ and R² groups, said groups being H or an alkaline cation (for R¹) or an alkaline cation (for R²).

6. The method according to claim 5 **characterized in that**, at the fifth step, the replacement of the R'¹ and R^{'2} groups of straight-chain alkyl type by a cation K⁺, Na⁺ or Li⁺ is performed with KOH, NaOH or LiOH respectively in a polar solvent, at a temperature of between 20 and 100°C, or else using trimethylsilyl bromide in a solvent at ambient temperature, followed by base hydrolysis.

7. The method according to claim 5 **characterized in that**, at the fifth step, the replacement by H of R'¹ or R'² groups of branched alkyl type is conducted by reaction with trifluoroacetic acid in an aprotic organic solvent.

8. A complex of lanthanide or a transition metal, **characterized in that** it comprises an ion of lanthanide or of a transition metal complexed with a ligand of formula (I) such as defined in any of claims 1 to 4.

9. The complex according to claim 8, **characterized in that** the lanthanide ion is selected from among Gd³⁺, Lu³⁺, Eu³⁺, Tb³⁺, Dy³⁺, Sm³+, Er³⁺, Yb³⁺, Pr³⁺ and Nd³⁺ ions.

10. The complex according to claim 8, **characterized in that** the ion of a transition metal is selected from among Cu(II), Co(II), Mn(II or IV), Ni(II), Fe(III), Pd(II) and Pt(II) ions.

11. The use of a complex such as defined in any of claims 8 to 10, wherein the Z group of the complex ligand is a -COOH group, COONa group, an activated ester of *N*-hydroxysuccinimide ester or pentafluorophenol ester type, a Br, an I or an amine, for the marking of compounds carrying an amine, alcohol, thiol, carboxylic acid or activated ester group.

12. The use of a complex such as defined in any of claims 8 to 10, as luminescent marker for two-photon absorption.

13. The use of a complex such as defined in any of claims 8 to 10, wherein the terminal Z group of the ligand is a biotin group, for the recognition of avidin, streptavidin and neutravidin.
